# EUROPEAN PATENT APPLICATION

(11) **EP 4 819 054 A1**
(43) Date of publication of application: **07.10.2026**
(21) Application number: 26166146.6
(22) Date of filing: 19.03.2026
(51) Int. Cl.: G06T 12/10

(54) **RECONSTRUCTION OF A THREE-DIMENSIONAL IMAGE OF A PATIENT**

(30) Priority: 27.03.2025 GB 202504521
(71) Applicant: Elekta Limited, Crawley, West Sussex RH10 9BL (GB)
(72) Inventor: BAL, Matthieu, Crawley, RH10 9BL (GB); JOYCE, Thomas, Crawley, RH10 9BL (GB); STANCANELLO, Joseph, Crawley, RH10 9BL (GB)
(74) Representative: Haseltine Lake Kempner LLP

(57) **Abstract**

A method (100) for reconstruction of 3D image of a patient is disclosed. The method comprises obtaining a projection set comprising a plurality of 2D CBCT projections of a first volume of the patient (110), and obtaining a 3D image of a second volume of the patient, wherein the second volume of the patient at least partially overlaps with the first volume of the patient (120). The method further comprises using acquisition information relating to the obtained projection set to generate, from the 3D image, synthetic projections of the second volume of the patient (130), and extending the projections of the first volume of the patient from the obtained projection set with information from the synthetic projections of the second volume of the patient (140). The method further comprises using the extended projections to generate a 3D image of a third volume of the patient, the third volume comprising a combination of the first and second volumes.

## Description

### Technical field

The present disclosure relates to a method for reconstruction of a three-dimensional (3D) image of a patient. The method may be performed by a reconstruction node, and the present disclosure also relates to a reconstruction node, a radiotherapy apparatus and a computer program product configured to execute a method for reconstruction of a 3D image of a patient.

### Background

Radiotherapy (RT), one of the cornerstones of cancer treatment, is the use of ionising radiation to damage or destroy unhealthy cells in a human or animal body. During treatment, the ionising radiation is formed into a beam and directed to unhealthy cells in the body, such as a tumour. The treatment planning procedure for RT may include using a three-dimensional image of the patient to identify a target region that comprises the tumour, and to identify organs near the tumour, termed Organs at Risk (OARs). A treatment plan aims to ensure delivery of a required dose of radiation to the tumour, while minimising the radiation dose to nearby OARs. The treatment plan for a patient is typically generated using medical images that have been obtained according to one or more imaging techniques, such as Magnetic Resonance Imaging (MRI) and Computed Tomography (CT). The radiation treatment plan includes parameters specifying the direction, cross sectional shape, and intensity of each radiation beam to be applied to the patient. The radiation treatment plan may include dose fractioning, in which a sequence of radiation treatments is provided over a predetermined period of time, with each treatment delivering a specified fraction of the total prescribed dose. For example, the total radiation dose may be divided into around 3 to 40 daily fractions.

Image guided RT (IGRT) is a technique in which images of the patient are acquired during the course of radiotherapy and used to guide the RT treatment, thereby increasing the accuracy of the treatment. For example, the images may be obtained at the time of fraction delivery using in-room imaging (e.g., Cone Beam Computed Tomography (CBCT) or MRI) and used to correctly align the planned dose distribution with the patient's anatomy. Another example of IGRT is adaptive radiotherapy (ART) in which the images of the patient are used to evaluate and adapt the treatment plan. The treatment plan may be adapted offline using scheduled imaging between dose fractions to detect changes that occur during the treatment course. This is referred to as inter-fraction adaptation. Adaptation may also be conducted online using same day imaging captured immediately prior to radiation delivery, while the patient is in the treatment position (another example of inter-fraction adaptation). Finally, real-time ART involves treatment plan adaptations during delivery of a radiation dose fraction, and is referred to as intra-fraction adaptation.

CBCT scanning lends itself to IGRT owing to its comparatively low imaging radiation dose, comparatively short scan time, and its facility of integration into an RT treatment apparatus comprising a linear accelerator. CBCT imaging for IGRT uses a cone shaped X-ray beam and flat panel detector. The kilovoltage source and detector rotate around a patient positioned on the RT treatment table. Despite its advantages, CBCT imaging typically results in significantly lower image quality owing to increased radiation scatter when compared with other imaging methods including fan-beam (planning) CT or Magnetic Resonance (MR) imaging. In addition, as discussed below, CBCT imaging offers a significantly smaller Field of View (FOV) than CT or MR imaging.

Multiple factors may impact the quality and completeness of the 3D image reconstructed from projections acquired using in-room imaging systems such as CBCT. For example, depending on the patient's size and/or position in the scanner, and the detector offset used when acquiring the patient volume, some parts of the patient might not be visible in the reconstructed FOV from the acquired projections. Frequently, it may be that the patient outline, or lateralized portions of the patient volume, are not visible in the reconstructed FOV. In some examples the FOV, being centred on the target volume, may not include anatomical structures which although distant from the target volume may still be at risk, or may be relevant for tasks such as segmentation, dose calculation or plan adaptation. The problem can be thought of as the acquired projections being 'too short', and thus missing parts of the anatomy that are not then represented in the reconstructed image. In addition to the challenges of parts of the patient anatomy being missing from the reconstructed image, truncation artifacts present in the limited FOV of the reconstruction negatively impact the overall image quality.

Various approaches have been proposed to extend the FOV of reconstructed patient images. One such approach, disclosed in Macfarlane M, Wong D, Hoover DA, Wong E, Johnson C, Battista JJ, et al. Patient-specific calibration of cone-beam computed tomography data sets for radiotherapy dose calculations and treatment plan assessment. J Appl Clin Med Phys 2018;19(2):249-57, involves registering a reconstructed CBCT volume with a planning (fan beam) CT volume having a larger FOV, before merging the resulting volumes in image space into a single anatomically complete CBCT volume. Other approaches seek to exploit the possibilities of Machine Learning (ML) models, for example by training a neural network on data from a population of patients to be able to correct truncated reconstructions in image space, or to extend the acquired projections so as to enable a larger reconstructed FOV.

### Summary

It is an aim of the present disclosure to at least partially address one or more of the challenges mentioned above. The invention is defined in the independent claims, to which reference should now be made. Further features are set out in the dependent claims.

According to a first aspect of the present disclosure, there is provided a computer-implemented method for reconstruction of a three-dimensional (3D) image of a patient. The method comprises obtaining a projection set comprising a plurality of two-dimensional (2D) projections of a first volume of the patient. The projections are acquired using a first apparatus which is a Cone-Beam Computed Tomography (CBCT) apparatus. The method further comprises obtaining a 3D image of a second volume of the patient. The 3D image is reconstructed from projections acquired using a second apparatus. The second volume of the patient at least partially overlaps with the first volume of the patient. The method further comprises using acquisition information relating to the obtained projection set to generate, from the obtained 3D image of the second volume of the patient, synthetic projections of the second volume of the patient. The acquisition information may for example comprise the acquisition geometry of the first apparatus, and may further comprise acquisition parameters of the projections in the obtained projection set. The method further comprises extending the projections of the first volume of the patient from the obtained projection set with information from the synthetic projections of the second volume of the patient. The method further comprises using the extended projections to generate a 3D image of a third volume of the patient, the third volume of the patient comprising a combination of the first volume of the patient and the second volume of the patient.

According to another aspect of the present disclosure, there is provided a management node for reconstruction of a three-dimensional (3D) image of a patient. The management node comprises processing circuitry configured to cause the management node to obtain a projection set comprising a plurality of two-dimensional (2D) projections of a first volume of the patient, the projections being acquired using a first apparatus which is a Cone-Beam Computed Tomography (CBCT) apparatus. The processing circuitry is further configured to cause the management node to obtain a 3D image of a second volume of the patient, the 3D image reconstructed from projections acquired using a second apparatus. The second volume of the patient at least partially overlaps with the first volume of the patient. The processing circuitry is further configured to cause the management node to use acquisition information relating to the obtained projection set to generate, from the obtained 3D image of the second volume of the patient, synthetic projections of the second volume of the patient. The acquisition information may in some examples comprise the acquisition geometry of the first apparatus, and may further comprise acquisition parameters of the projections in the obtained projection set. The processing circuitry is further configured to cause the management node to extend the projections of the first volume of the patient from the obtained projection set with information from the synthetic projections of the second volume of the patient. The processing circuitry is further configured to cause the management node to use the extended projections to generate a 3D image of a third volume of the patient. The third volume of the patient comprises a combination of the first volume of the patient and the second volume of the patient.

According to another aspect of the present disclosure, there is provided a computer program product comprising a computer readable medium, the computer readable medium having computer readable code embodied therein, the computer readable code being configured such that, on execution by a suitable computer or processor, the computer or processor is caused to perform a method according to any one or more aspects or examples of the present disclosure.

According to another aspect of the present disclosure, there is provided a radiotherapy treatment apparatus comprising a management node according to the present disclosure.

Examples of the present disclosure therefore provide improved methods and nodes for reconstructing a 3D image of a patient. Example methods according to the present disclosure use both a CBCT projection set and a separate 3D image, the patient volumes represented by the projection set and 3D image being at least partially overlapping. In this manner, example methods may leverage anatomical prior patient information present in the 3D image to extend the projections of the CBCT projection set, and so reconstruct a new 3D image of the patient having a larger FOV. It will be appreciated that example methods disclosed herein extend the FOV by working in projection space, extending projections of the CBCT projection set before then generating a final reconstructed 3D image. By working in projection space to extend the CBCT projection data, as opposed for example to correcting a reconstructed volume, example methods according to the present disclosure can mitigate truncation artefacts that are frequently present in limited FOV reconstructed CBCT images, thus improving overall image quality.

Examples according to the present disclosure may be implemented in digital electronic circuitry, or in computer hardware, firmware, software, or in combinations thereof. Examples of the disclosure may be implemented as a computer program or a computer program product, i.e., a computer program tangibly embodied in a non-transitory information carrier, e.g., in a machine-readable storage device or in a propagated signal, for execution by, or to control the operation of, one or more hardware modules. A computer program may be in the form of a stand-alone program, a computer program portion, or more than one computer program, and may be written in any form of programming language, including compiled or interpreted languages, and it may be deployed in any form, including as a stand-alone program or as a module, component, subroutine, or other unit suitable for use in a data processing environment.

The disclosure is set out herein in terms of particular examples. Other examples, not explicitly described here, may nonetheless fall within the scope of the claims. Unless explicitly or implicitly specified otherwise, the steps of methods according to examples of the disclosure may be performed in a different order and still achieve desirable results.

### Brief description of the drawings

For a better understanding of the present disclosure, and to show more clearly how it may be carried into effect, reference will now be made, by way of example, to the following drawings in which:
Figure 1 is a flow chart illustrating process steps in a method for reconstruction of a three-dimensional (3D) image of a patient;
Figures 2a to 2f show flow charts illustrating further examples of methods for reconstruction of a 3D image of a patient;
Figure 3 is a block diagram illustrating a radiotherapy system suitable for reconstruction of a 3D image of a patient;
Figure 4 illustrates a high-level schematic of an example implementation of the methods disclosed herein;
Figure 5 is a flow diagram illustrating steps in an implementation of methods disclosed herein;
Figure 6 is a flow diagram illustrating steps in another implementation of methods disclosed herein;
Figure 7 illustrates a comparison between a reconstructed CBCT image, and a reconstructed CBCT image that has been extended according to methods of the present disclosure;
Figure 8 illustrates the geometry of a typical cone beam projection acquisition setup;
Figure 9 illustrates the geometry of a cone beam projection acquisition setup, as incorporated into a medical CT scanner;
Figure 10 depicts a radiotherapy apparatus; and
Figure 11 is a block diagram of an implementation of a radiotherapy system.

### Detailed description of examples

Examples of the present disclosure propose methods enabling the extension of the field of view (FOV) of an image of a patient obtained using Cone Beam Computed Tomography (CBCT).

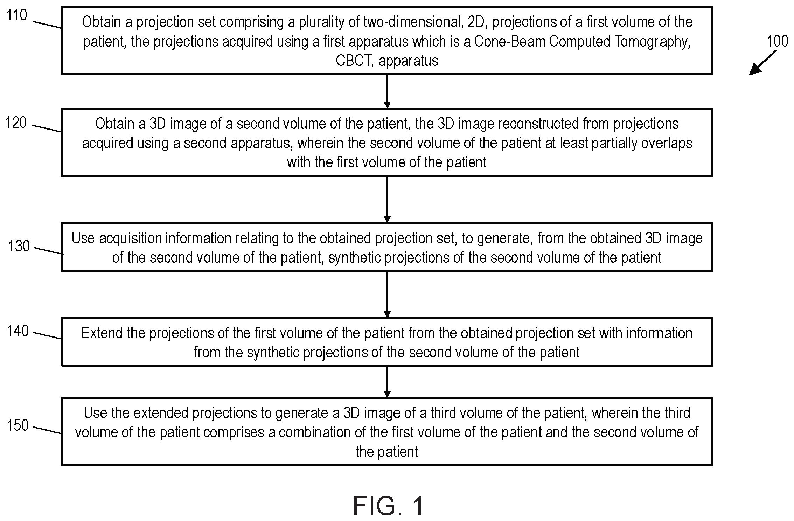

Figure 1 is a flow chart showing process steps in a computer-implemented method 100 for reconstruction of a 3D image of a patient according to examples of the present disclosure. The method 100 may for example be used in connection with the planning and/or delivery of IGRT.

Referring to Figure 1, in step 110, the method 200 comprises obtaining a projection set comprising a plurality of two-dimensional (2D) projections of a first volume of the patient. The first volume of the patient may for example include a Clinical Target Volume (CTV) or Planning Target Volume (PTV) of the patient. CTV and PTV include a tumour volume as well as a margin for disease spread that cannot be imaged (CTV), and margin allowing for uncertainties in planning or treatment delivery (PTV). The projections of the obtained projection set are acquired using a first apparatus which is a CBCT apparatus. As discussed above with reference to CBCT imaging, the obtained projections et may enable reconstruction of a 3D image that offers a relatively limited FOV, encompassing just the target volume and immediate surroundings, but for example not including lateral portions of the patient, or remote tissue structures. In some examples, the obtained projection set may be have been acquired during a CBCT scan of the patient prior to a delivery of a daily dose fraction of radiotherapy.

In step 120, the method 100 comprises obtaining a 3D image of a second volume of the patient. The 3D image is reconstructed from projections acquired using a second apparatus. The second volume of the patient at least partially overlaps with the first volume of the patient. The second volume of the patient may in different examples be a smaller volume, the same size volume or a larger volume than the first volume of the patient. In some examples, the second volume of the patient may entirely contain within its volume the first volume of the patient. As discussed in greater detail below, the second apparatus may comprise a Fan-Beam CT apparatus, an MRI apparatus and/or a CBCT apparatus. In examples in which the second apparatus comprises a Fan-Beam CT apparatus, the 3D image obtained at step 120 may comprise a panning CT of the patient.

In step 130, the method 100 comprises using acquisition information relating to the obtained projection set to generate, from the obtained 3D image of the second volume of the patient, synthetic projections of the second volume of the patient. According to different examples of the present disclosure, the acquisition information may comprise the acquisition geometry of the first apparatus, and may also comprise acquisition parameters of the projections in the obtained projection set.

In step 140, the method 100 comprises extending the projections of the first volume of the patient from the obtained projection set with information from the synthetic projections of the second volume of the patient. Various approaches may be used to extend the projections of the first volume of the patient from the obtained projection set with information from the synthetic projections of the second volume of the patient, as discussed in greater detail below with reference to the method 200. These approaches may include a heuristic approach, and/or use of one or mode Machine Learning (ML) models.

In step 150, the method 100 comprises using the extended projections to generate a 3D image of a third volume of the patient. As illustrated in Figure 1, the third volume of the patient comprises a combination of the first volume of the patient and the second volume of the patient. In some examples, the combination of the first volume and the second volume may comprise all of the first volume and at least some of the second volume. In further examples, the combination of the first volume and the second volume may comprise all of the first volume and all of the second volume. In examples in which the first volume is entirely contained within the second volume, the combination of the first volume and the second volume may comprise the second volume. It will be appreciated that, owing to the use of the extended projections to generate the 3D image of the third patient volume, the FOV of the 3D image of the third volume will be greater than the FOV that would be obtained by reconstructing a 3D image using only the projections of the CBCT projection set obtained in step 110 of the method 100.

Examples of the method 100 thus leverage patient specific information from the 3D image obtained in step 120 (which could be a planning CT image, an MR image, or previous CBCT image) to extend the FOV that would otherwise be available from reconstruction of projections in the obtained CBCT projection set. In addition, it will be appreciated that example of the method 100 operate to extend the FOV by acting in the projection space, manipulating projections as opposed to reconstructed volumetric images. The final reconstructed image of the third volume may be used for example in downstream RT tasks, such as treatment plan adaptation. By including a larger FOV in the image, treatment plan adaptation may take into account more distant tissue structures, allowing for the evaluation of a greater range of treatment plan variables.

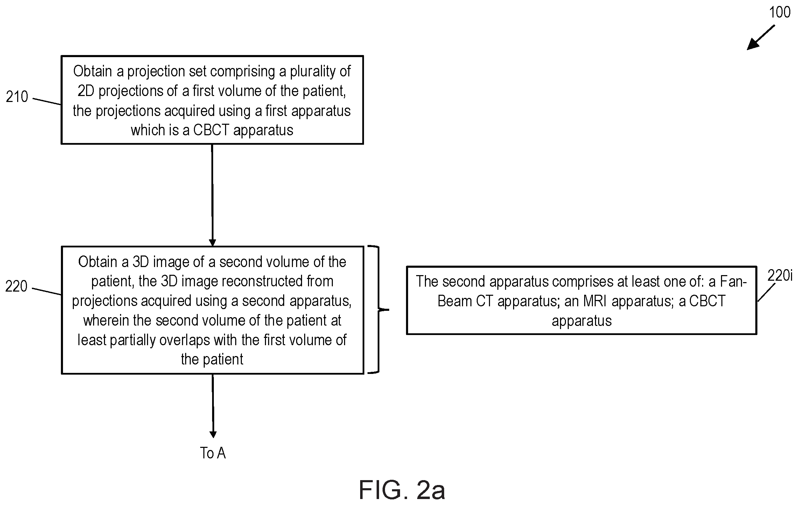

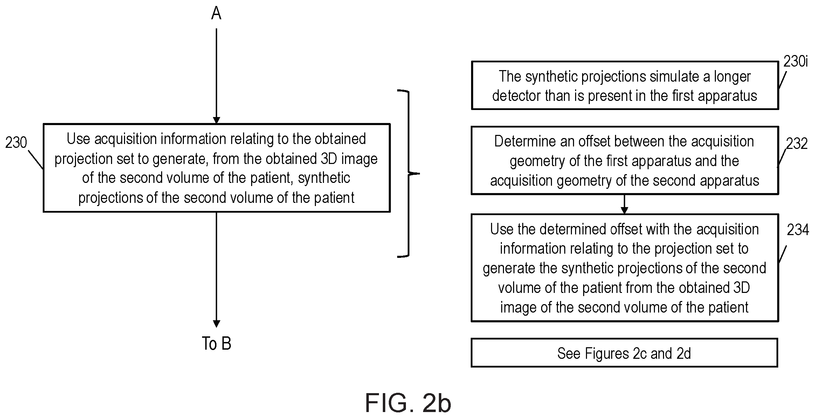

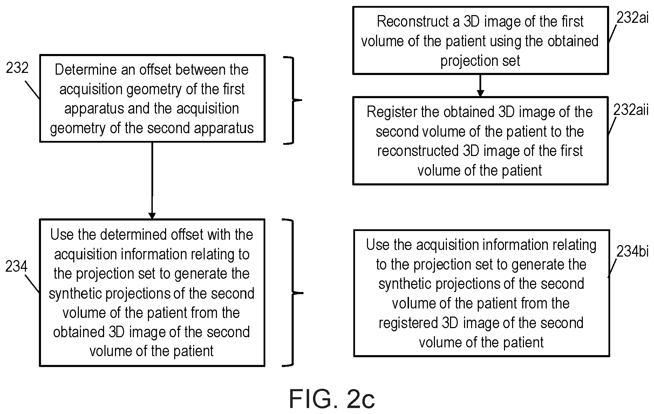

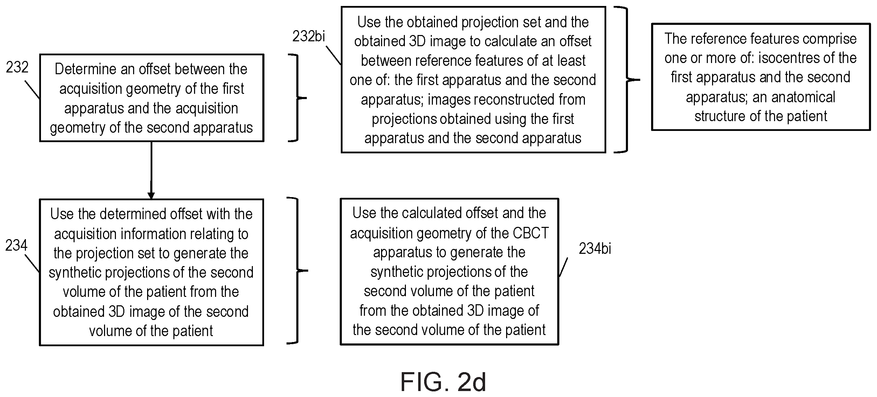

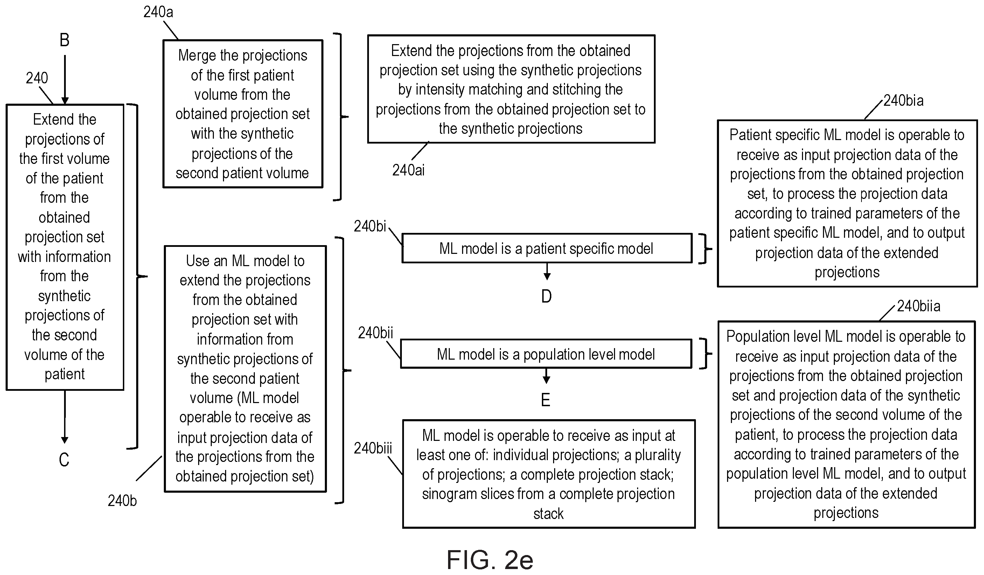

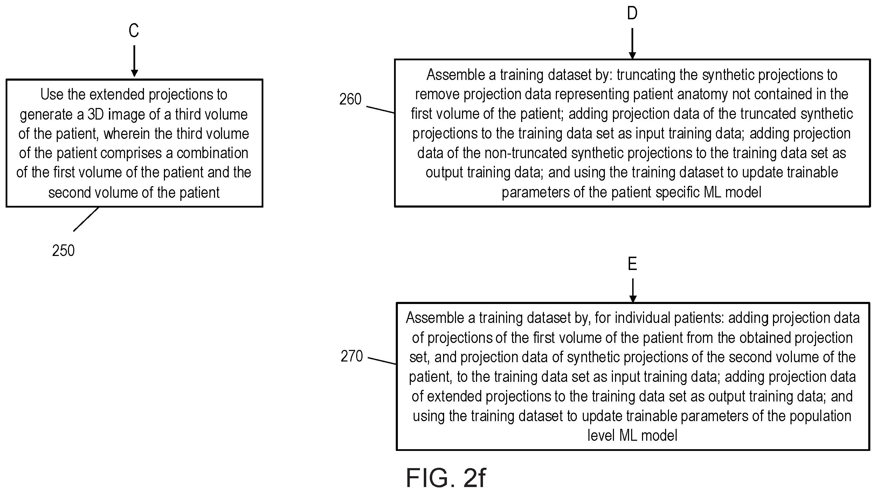

Figure 2 is a flow chart showing process steps in another example of a computer-implemented method 200 for reconstruction of a 3D image of a patient according to examples of the present disclosure. As for the method 100 discussed above, the method 200 may be performed by a reconstruction node, and may be used in connection with the planning and/or delivery of IGRT. The method 200 illustrates examples of how the steps of the method 100 may be implemented and supplemented to provide the above discussed and additional functionality.

Referring initially to Figure 2a, in a first step 210, the reconstruction node performing the method 200 obtains a projection set comprising a plurality of 2D projections of a first volume of the patient. As illustrated in Figure 2a, the projections have been acquired using a first apparatus which is a CBCT apparatus. In step 220, the reconstruction node then obtains a 3D image of a second volume of the patient. Again as illustrated in Figure 2a, the 3D image is reconstructed from projections acquired using a second apparatus, and the second volume of the patient at least partially overlaps with the first volume of the patient. In some examples, as illustrated at 220i, the second apparatus may comprise at least one of: a Fan-Beam CT, apparatus, an MR imaging apparatus, and/or a CBCT apparatus. In examples in which the second apparatus is a CBCT apparatus, in some examples the second apparatus may comprise the same CBCT apparatus as the first apparatus. In other examples, the second apparatus may comprise a CBCT apparatus that is different to the first apparatus. It will be appreciated that a Fan Beam CT apparatus and MR apparatus offer a naturally larger FOV than a CBCT apparatus. Consequently, 3D images reconstructed from projections acquired by such apparatus may represent a second volume that is larger than the first volume. In such examples, the second volume may partially overlap with the first volume, such that at least a part of the first volume is also included in the second volume, or the second volume may completely encompass the first volume, such that all of the first volume is also included in the second volume. In contrast, if the second apparatus is a CBCT apparatus, then the second volume represented by the obtained 3D image may be substantially the same size as the first volume. In such examples, it is more likely that the first and second volumes will partially overlap, ensuring that the second volume contains tissue structures that are not contained in the first volume.

Referring now to Figure 2b, in step 230, the reconstruction node uses acquisition information relating to the obtained projection set to generate, from the obtained 3D image of the second volume of the patient, synthetic projections of the second volume of the patient. In some examples of the present disclosure, the acquisition information may comprise the acquisition geometry of the first apparatus, and the acquisition information may also comprise acquisition parameters of the projections in the obtained projection set. As illustrated at 230i, the generated synthetic projections may simulate a longer detector than is present in the first apparatus. Hence, in some examples, the synthetic projections may represent projections that would have resulted in the obtained 3D image of the second volume of the patient, had those projections been acquired using acquisition information relating to the CBCT apparatus modified such that the detector panel is longer than is the case for the actual CBCT apparatus that was used as the first apparatus.

In some examples, as illustrated in Figure 2b, the step 230 of generating synthetic projections of the second volume of the patient may comprise, in step 232, determining an offset between the acquisition geometry of the first apparatus and the acquisition geometry of the second apparatus, and in step 234, using the determined offset with the acquisition information relating to the projection set to generate the synthetic projections of the second volume of the patient from the obtained 3D image of the second volume of the patient. Figures 2c and 2d illustrate different example ways in which the steps 232 and 234 may be implemented.

Referring initially to Figure 2c, in a first example (a), determining an offset between the acquisition geometry of the first apparatus and the acquisition geometry of the second apparatus may comprise first reconstructing a 3D image of the first volume of the patient using the obtained projection set in step 232ai, and then registering the obtained 3D image of the second volume of the patient to the reconstructed 3D image of the first volume of the patient in step 132aii. Continuing the first example (a), if the offset is determined in the manner of steps 132ai and 132aii, then using the determined offset with the acquisition information relating to the projection set to generate the synthetic projections of the second volume of the patient from the obtained 3D image of the second volume of the patient may comprise performing step 234ai. Step 134ai comprises using the acquisition information relating to the projection set to generate the synthetic projections of the second volume of the patient from the registered 3D image of the second volume of the patient.

Referring now to Figure 2d, in a second example (b), determining an offset between the acquisition geometry of the first apparatus and the acquisition geometry of the second apparatus may comprise, in step 132bi, using the obtained projection set and the obtained 3D image to calculate an offset between reference features of at least one of the first apparatus and the second apparatus, and/or images reconstructed from projections obtained using the first apparatus and the second apparatus. As illustrated in Figure 2d, the reference features may comprise one or more of: isocentres of the first apparatus and the second apparatus, and/or an anatomical structure of the patient. In some examples, the anatomical structure of the patient may comprise a bone or bone structure of the patient, an organ of the patient and/or any other soft tissue structure of the patient. Continuing the second example (b), if the offset is determined in the manner of step 132bi, then using the determined offset with the acquisition information relating to the projection set to generate the synthetic projections of the second volume of the patient from the obtained 3D image of the second volume of the patient may comprise performing step 234bi. Step 134bi comprises using the calculated offset and the acquisition geometry of the CBCT apparatus to generate the synthetic projections of the second volume of the patient from the obtained 3D image of the second volume of the patient.

Referring now to Figure 2e, following generation of the synthetic projections of the second volume in step 230, the reconstruction node then, in step 340, extends the projections of the first volume of the patient from the obtained projection set with information from the synthetic projections of the second volume of the patient. These extended projections are then used by the reconstruction node in step 250 (as illustrated in Figure 2f) to generate a 3D image of a third volume of the patient, the third volume of the patient comprising a combination of the first volume of the patient and the second volume of the patient.

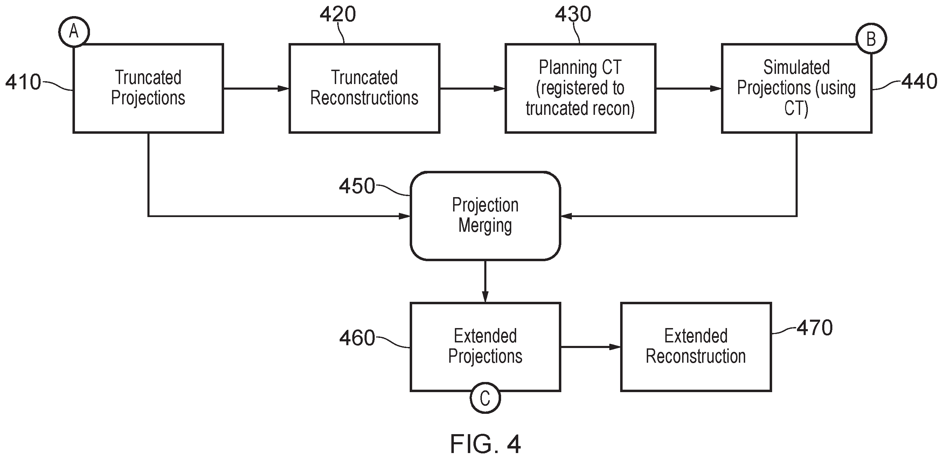

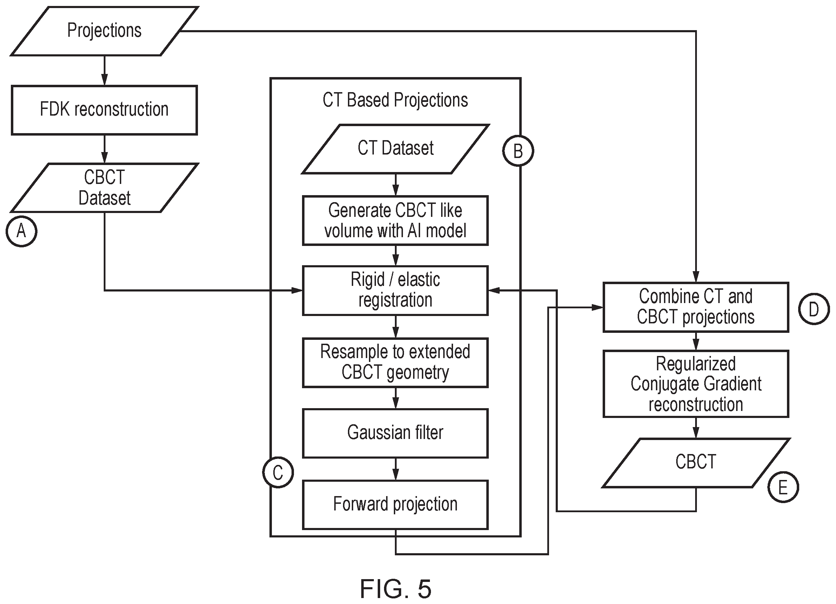

Step 240 of extending the projections of the first volume of the patient from the obtained projection set with information from the synthetic projections of the second volume of the patient may be performed using a variety of different approaches, three of which are illustrated in Figures 2e and 2f. Example implementations of these approaches are discussed in detail later in the present disclosure, with reference to Figures 4 and 5.

Referring again to Figure 2e, in a first approach to performing step 240 (extending the projections of the first volume), the reconstruction node may, in step 240a, merge the projections of the first volume of the patient from the obtained projection set with the synthetic projections of the second volume of the patient. This may be considered as a heuristic approach to the step of extending the CBCT projections of the first volume. In some examples, as illustrated at 240ai, merging the projections of the first volume of the patient from the obtained projection set with the synthetic projections of the second volume of the patient may comprise extending the projections of the first volume of the patient from the obtained projection set using the synthetic projections of the second volume of the patient. This extending may be performed by intensity matching and stitching the projections of the first volume of the patient from the obtained projection set to the synthetic projections of the second volume of the patient. Various additional processing steps may be performed to enhance the intensity matching and stitching, as discussed later in the present disclosure with reference to Figure 4.

Referring still to Figure 2e, second and third approaches to performing step 240 (extending the projections of the first volume) comprise the use of an ML model. As illustrated at 240b, in the second and third approaches, extending the projections of the first volume of the patient with information from the synthetic projections of the second volume of the patient comprises using an ML model to extend the projections of the first volume of the patient from the obtained projection set with information from the synthetic projections of the second volume of the patient. As illustrated at 240b, the ML model is operable to receive as input projection data of the projections of the first volume of the patient from the obtained projection set. The ML model may comprise an artificial neural network (ANN), or any other suitable network architecture. It will be appreciated that, for a given projection of the first volume from the projection set, many plausible and realistic extended versions may exist. It is therefore desirable to provide guidance to the ML model so as to ensure that the output of the ML model (the extended projections of the first volume) is as close as possible to a realistic representation of the specific patient concerned. The second and third approaches illustrated in Figures 2e and 2f represent two possibilities for ensuring this correct correspondence of the ML model output to the patient. The second approach uses a patient specific ML model, and the third approach sues a population level ML model.

In the second approach, as illustrated at step 240bi, the ML model is a patient specific model. According to examples of the present disclosure, this implies that the ML model is trained using training data that is specific to the patient, and so the model is adapted specifically to make predictions that are representative of the patient. The patient specific approach implies training an ML model may be trained for each patient undergoing treatment, as discussed in greater detail below. As illustrated at 240bia, the patient specific ML model may be operable to receive as input projection data of the projections of the first volume of the patient from the obtained projection set, to process the projection data according to trained parameters of the patient specific ML model, and to output projection data of the extended projections. In some examples, in order to produce an ML model that extends the projections in an anatomically realistic way for the specific patient being considered, the patient specific model may be trained using only the synthetic projections created at step 230 from the obtained 3D image of the patient. The obtained 3D image may in one example be a planning CT of the patient, and, as discussed above, the synthetic projections may be representative of projections that would have resulted in the obtained 3D image of the second volume of the patient, had those projections been acquired using acquisition information relating to the CBCT apparatus modified such that the detector panel is longer than is the case for the actual CBCT apparatus that was used as the first apparatus. The synthetic projections thus contain the appropriate anatomical information of the specific patient under consideration, and can be used to generate training data that will train the ML model to extend the CBCT projections of the projection set in a manner that is anatomically correct for the patient, effectively predicting the anatomy of those regions of the second patient volume that are not present in the first volume.

Referring now to Figure 2f, and in some examples, use of a patient specific ML model at step 240b and 240bi may further comprise assembling a training data set for the ML model, and using the training dataset to train the patient specific ML model, in step 260. In some examples, particularly as concerns the patient specific ML model, the step 260 of assembling a training dataset and training the patient specific ML model may be carried out before use of the model to extend projections at step 240. As illustrated at 260, assembling the training dataset may comprise truncating the synthetic projections to remove projection data representing patient anatomy not contained in the first volume of the patient, and adding projection data of the truncated synthetic projections to the training data set as input training data. Assembling the training dataset may further comprise adding projection data of the non-truncated synthetic projections to the training data set as output training data. Training the patient specific ML model may then comprise using the training dataset to update trainable parameters of the patient specific ML model, such that the patient specific ML model is trained to generate, for an input comprising truncated synthetic projection data, predicted extended projection data that resembles the corresponding non-truncated synthetic projection data. At inference, the patient specific ML model will thus be operable to predict extended versions of the CBCT projections of the first patient volume that are input to the patient specific ML model.

In some examples, the patient specific ML model may be trained once for a given specific patient at the first fraction. The trained patient specific ML model may then be used for inference at each subsequent fraction. In another example, the training of the patient specific ML model may be undertaken for each fraction. In a still further example, these approached may be combined, with training of the patient specific ML model carried out at the first fraction, and then finetuning of the patient specific ML model conducted at subsequent fractions.

Referring again to Figure 2e, in the third approach, as illustrated at step 240bii, the ML model is a population level model. According to examples of the present disclosure, this implies that the ML model is trained using training data that is drawn from a population of multiple patients. In some examples, the plurality of patients that comprise the population may include a large variety of patient types. In one example, patient types may include patients that are underweight or overweight. In other examples, patient types may include patients that have or have not undergone surgery or other procedures. Surgery may include for example the posing of implantable prostheses such as replacement joints, removal of internal organs such as the uterus, or other procedures. Other patient types may be envisaged. The training data for the population level ML model may utilise data from all of the individual patients in the population. In other examples, the training data for the population level ML model may utilise data from a subset of the individual patients in the population. It will be appreciated that the population level ML model is trained to predict extended projections on the basis of anatomical features that are common to all patients in the population. Consequently, the population level ML model may be provided at inference with more information about the specific patient under consideration, to ensure that the predicted extended projections are representative of that patient. As illustrated at 240biia, the population level ML model may be operable to receive as input projection data of the projections of the first volume of the patient from the obtained projection set, and projection data of the synthetic projections of the second volume of the patient. The population level model may then be operable to process the projection data according to trained parameters of the population level ML model, and to output projection data of the extended projections.

It will be appreciated that, as discussed above, the population level ML model learns to predict extended projections based on anatomical features that are common across the population from which training data for the model was obtained. However, the model does not contain any encoding or representation of the specificities of any given patient in the population. Consequently, the population level model receives as input, in addition to the CBCT projections of the first patient volume, the synthetic projections of the second patient volume. The synthetic projections of the second patient volume provide the model with information about the specific anatomy of the patient under consideration, enabling the model to generate a representative prediction for that particular patient using both the information about anatomical features common to the population that is encoded in the model during its training, and information about how that information is appropriate for the specificities of this particular patient taken from the synthetic projections of the patient that are input to the ML model.

Referring again to Figure 2f, and in some examples, use of a population level ML model at step 240b and 240bii may further comprise assembling a training data set for the ML model, and using the training dataset to train the patient specific ML model, in step 270. In some examples, the step 260 of assembling a training dataset and training the patient specific ML model may be carried out at different times. For example, training data relating to individual patients may be added to the training dataset during performance of the method 200 for such patients, and the population level model may be periodically trained and/or retrained using the assembled training dataset. In other examples, previously generated and stored training data may be used for initial training of the population level ML model. As illustrated at 270, assembling the training dataset may comprise adding projection data of projections of the first volume of the patient from the obtained projection set, and projection data of synthetic projections of the second volume of the patient, to the training data set as input training data. Assembling the training dataset may further comprise adding projection data of extended projections to the training data set as output training data. Training the population level ML model may then comprise using the training dataset to update trainable parameters of the population level ML model, such that the population level ML model is trained to generate, for an input comprising truncated synthetic projection data and non-truncated synthetic data, predicted extended projection data that corresponds to the patient anatomy represented in the input projection data. During inference, the model can then predict extended projection data using an input of CBCT projections and synthetic projections generated from the obtained 3D image.

It will be appreciated that in some examples, to generate the extended projection data that is used as output training data for training the population level ML model, the first (heuristic) approach discussed above may be used, comprising intensity matching and direct stitching of the obtained projection set and the synthetic projections of the individual patients to generate the target extended projections used as output training data.

Referring again to Figure 2e, for both the patient specific ML model and population level ML model, as illustrated at 240biii, the input projection data may be provided in the form of at least one of individual projections, a plurality of projections, a complete projection stack and/or sinogram slices from a complete projection stack. The sinogram slices may be a part of a sinogram dataset. Thus, either the patient specific ML model or the population level ML model may be trained in 2D (using either single projections or sinogram slices from the 3D projection stacks), or in 3D on groups of projections or the 3D projection stack as a whole. In the 3D approach, the ML model may use all projection data to predict the extension of a given projection.

Example methods according to the present disclosure thus enable generation of a reconstructed image from a CBCT scan that has an enlarged FOV (representing a combination of both the first and second patient volumes) with respect to what would be possible by reconstruction from the CBCT projections alone (representing only the first volume). Example methods disclosed herein leverage prior information about the patient present in an obtained 3D image, such as the patient planning CT, a planning MRI, and/or a prior CBCT image. In this manner, example methods described herein offer a patient specific approach, in which extension of the FOV is based on information from the relevant patient, and not just generic anatomical information applicable to a population of patients. In addition, example methods disclosed herein operate in projection space, extending the existing real projection data, and so mitigating truncation artifacts that are frequently present in CBCT reconstructions. It will be appreciated that leveraging the prior patient information in projection space additionally achieves extension of FOV of the reconstructed image whilst potentially also improving the internal consistency of the projection data, and thus improving the resulting reconstruction even within non-truncated regions.

The extended FOV in the reconstructed image achieved by example methods disclosed herein can support both the planning and delivery of radiotherapy treatment. In one particular use case for methods of the present disclosure, a dose from a previous treatment session can be deformed or modified in light of the reconstructed image of the combination of the first and second patient volumes. The output of the methods disclosed herein may thus be used in the creation or adaptation of a radiotherapy treatment plan.

As discussed above, the methods 100 and 200 may be performed by a reconstruction node, and the present disclosure provides a reconstruction node that is adapted to perform any or all of the steps of the above discussed methods. The reconstruction node may comprise a physical or virtual node, and may be implemented in a computer system, treatment apparatus, computing device, or server apparatus, and/or may be implemented in a virtualized environment, for example in a cloud, edge cloud, or fog deployment. Examples of a virtual node may include a piece of software or computer program, a code fragment operable to implement a computer program, a virtualised function, or any other logical entity. The reconstruction node may encompass multiple logical entities, as discussed in greater detail below.

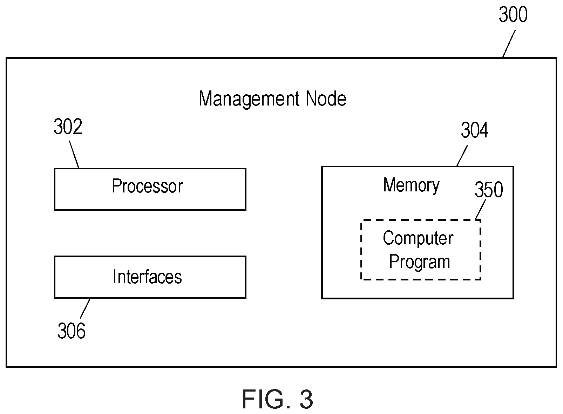

Figure 3 is a block diagram illustrating an example reconstruction node 300 which may implement the method 100 and/or 200, as illustrated in Figures 1 and 2a to 2f, according to examples of the present disclosure, for example on receipt of suitable instructions from a computer program 350. Referring to Figure 3 the reconstruction node 300 comprises a processor or processing circuitry 302, and may comprise a memory 304 and interfaces 306. The processing circuitry 302 is operable to perform some or all of the steps of the method 100 and/or 200 as discussed above with reference to Figures 1 and 2a to 2f. The memory 304 may contain instructions executable by the processing circuitry 302 such that the reconstruction node 300 is operable to perform some or all of the steps of the method 100 and/or 200, as illustrated in Figures 1 and 2a to 2f. The instructions may also include instructions for executing one or more telecommunications and/or data communications protocols. The instructions may be stored in the form of the computer program 350. In some examples, the processor or processing circuitry 302 may include one or more microprocessors or microcontrollers, as well as other digital hardware, which may include digital signal processors (DSPs), special-purpose digital logic, etc. The processor or processing circuitry 302 may be implemented by any type of integrated circuit, such as an Application Specific Integrated Circuit (ASIC), Field Programmable Gate Array (FPGA) etc. The memory 304 may include one or several types of memory suitable for the processor, such as read-only memory (ROM), random-access memory, cache memory, flash memory devices, optical storage devices, solid state disk, hard disk drive, etc.

In some examples as discussed above, the reconstruction node may be incorporated into treatment apparatus, and examples of the present disclosure also provide a treatment apparatus, such as a radiotherapy treatment apparatus, comprising either or both of a reconstruction node as discussed above and/or a planning node operable to implement a method for adapting a radiotherapy treatment plan.

Figures 1 to 2f discussed above provide an overview of methods which may be performed according to different examples of the present disclosure. These methods may be performed by a reconstruction node, as illustrated in Figure 3. There now follows a detailed discussion of how different process steps illustrated in Figures 1 to 2f and discussed above may be implemented. The functionality and implementation detail described below is discussed with reference to the modules of Figure 3 performing examples of the methods 100 and/or 200, substantially as described above.

Figure 4 illustrates a high-level schematic of an example implementation of the methods disclosed herein. Referring to Figure 4, following execution of steps 110, 210, 120, 220, the reconstruction node carrying out the method is has obtained 2D projections of the first volume of the patient (illustrated as truncated projections 410, owing to the limited FOV allowed by representation of only the first patient volume), and a 3D image of a second patient volume (illustrated in the implementation as the planning CT). As discussed above with respect to the methods 100 and 200, the second volume at least partially overlaps with the first volume. In the illustrated implementation, the second volume, imaged in the planning CT, will be larger than the first volume, owing to the larger FOV afforded by fan beam CT when compared with CBCT (used to acquire the 2D projections in step 110, 210).

In the illustrated implementation of Figure 4, the reconstruction node generates the synthetic projections of the second volume by performing steps 130, 230, 232, 232ai, 232aii, 234, and 234bi. That is, the reconstruction node reconstructs a 3D image of the first patient volume using the truncated projections (step 232ai, the outcome of which is illustrated at 420), and then registers the planning CT to the truncated reconstruction (step 232aii, the outcome of which is illustrated at 430). The reconstruction node then generates simulated projections of the second volume from the registered planning CT (step 234bi, the outcome of which is illustrated at 440). In an implementation of step 140, 240, the reconstruction node then extends the projections of the first volume of the patient from the obtained projection set with information from the synthetic projections of the second volume of the patient. In the illustrated implementation of Figure 4, this is achieved by performing step 240a of method 200 (illustrated at 450) of merging the truncated projections with the synthetic projections, resulting in extended projections 460. These extended projections are then used in an implementation of step 150, 250 to generate an extended 3D reconstruction 470. The extended 3D reconstruction 470 shows a third volume that is a combination of the first and second volumes. Owing to the use of the planning CT, the reconstructed image generated from the extended projections with offer a larger FOV than was afforded by the originally obtained CBCT projections.

As discussed above with reference to the methods 100 and 200, the step 140, 240 of extending the projections of the first volume of the patient from the obtained projection set with information from the synthetic projections of the second volume of the patient may be performed using a variety of approaches, with three approaches being described above. Briefly, these approaches can be considered as direct merging (intensity matching and direct stitching, i.e. a heuristic approach that takes A and B as inputs and returns C; a patient specific ML model that is trained (or finetuned) on B, then run on A to produce C; and a population level model which, at inference, takes A and B as inputs and returns C.

Figure 5 is a flow diagram illustrating steps in an implementation of the first approach discussed above, which comprises merging the projections of the first volume of the patient from the obtained projection set with the synthetic projections of the second volume of the patient, and may be considered as a heuristic approach to the step of extending the CBCT projections of the first volume. This approach is illustrated in Figure 2e in steps 240a and 240ai. Figure 5 also illustrates an implementation in which the reconstruction node generates the synthetic projections of the second volume by performing steps 130, 230, 232, 232ai, 232aii, 234, and 234bi (generating an initial reconstruction from the CBCT projections, and registering the planning CT to the CBCT reconstruction).

Referring now to Figure 5, the obtained 2D projection set (projections) from step 110, 210 are used to generate a first reconstructed volume (A) (step 232ai). In one example this reconstruction may be performed using an FDK reconstruction approach, but it will be appreciated that other reconstruction methods and approaches may be used. The planning CT volume (B) is then registered to this CBCT volume with either a rigid or an elastic deformation algorithm (step 232aii). In the illustrated implementation, before registration, an AI model is used to process the CT volume to cause it to more closely resemble a volume reconstructed from projections obtained using a CBCT apparatus: that is to cause the planning CT volume to be more "CBCT-like". Following registration, the registered CT volume is processed (for example using a Gaussian filter) to avoid artifacts arising from any remaining discrepancies between the CT and CBCT volumes (C). Forward projection is then used to obtain projection data from the registered and processed CT volume (steps 234, 234bi). In some examples, in order to reduce differences between the real CBCT projections and the synthetic projections generated from the registered planning CT volume, more realistic forward projection methods (including for example Monte Carlo based methods) may be used to generate the synthetic projections. These larger synthetic projections of the CT derived dataset are used to extend the real CBCT projections (D) (step 240), in this instance through direct intensity matching and stitching (steps 240a, 240ai). These extended projections can then be used to backproject into a volume with a larger field of view resulting in the extended CBCT volume (E) (step 250). As illustrated in Figure 5, by iterating between the backward projection and the extension of the projections, the difference between the CT and CBCT can be further reduced.

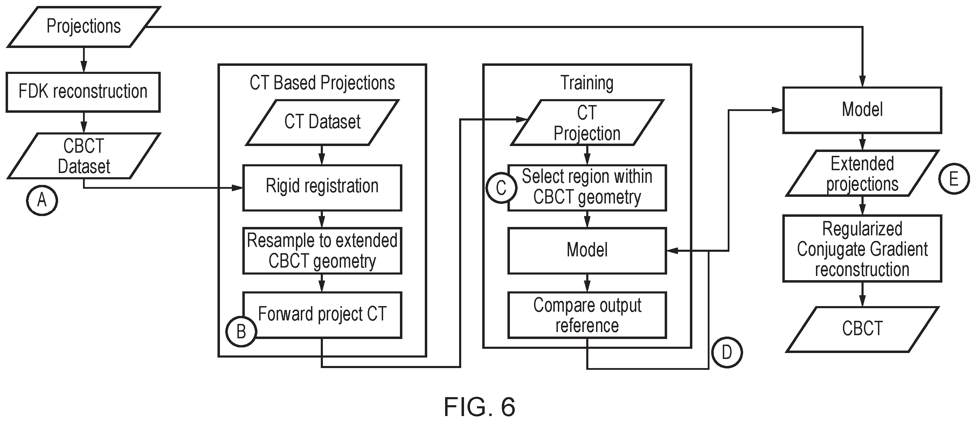

Figure 6 is a flow diagram illustrating steps in an implementation of the second approach to extending projections discussed above, in which a patient specific ML model is used to extend the projections of the first volume of the patient from the obtained projection set with information from the synthetic projections of the second volume of the patient. As discussed above, according to examples of the present disclosure, this implies that the ML model is trained using training data that is specific to the patient, and so the model is adapted specifically to make predictions that are representative of the patient. As for Figure 5, Figure 6 also illustrates an implementation in which the reconstruction node generates the synthetic projections of the second volume by performing steps 130, 230, 232, 232ai, 232aii, 234, and 234bi (generating an initial reconstruction from the CBCT projections, and registering the planning CT to the CBCT reconstruction).

As discussed above, the second (patient specific ML model) approach to extending the CBCT projections of the obtained projection set uses an ML model, such as a neural network, to predict those regions missing from the projection data by leveraging the obtained 3D image to spatially extend the acquired projection data. In the example illustrated in Figure 6, the obtained 3D image is the patient's planning CT, which contains all previously acquired relevant anatomy.

At a high level, both the second and third approaches involve training a neural network to extend the CBCT projections of the projection set. However, without additional information the problem is under-defined, as for a given short (CBCT) projection many plausible realistic longer versions could exist. The challenge is therefore to produce a network that extends the projections in an anatomically realistic way for the specific patient being considered. The third approach uses a population level ML model, and introduces patient specificity by submitting as input to the population level model both he CBCT projections and the patient specific synthetic projections (obtained at step 130, 230 of the methods described herein). The second, an example of which is illustrated in Figure 6, trains or finetunes an ML model using synthetic projections created using the patient specific planning CT as training output. As discussed above, the patient specific ML model could be trained in 2D (using either single projections or sinogram slices from the 3D projection stack), or on the 3D projection stack as a whole. In the 3D approach, the ML model can in some examples use all projection data to predict the extension of a given projection.

Referring now to Figure 6, the obtained 2D projection set (projections) from step 110, 210 are used to generate a first reconstructed volume (A) (step 232ai). In one example this reconstruction may be performed using an FDK reconstruction approach, but it will be appreciated that other reconstruction methods and approaches may be used. In some examples, the obtained 3D image (planning CT), may then be modified using a pre-trained model to more closely resemble an image reconstructed from projection data acquired using a CBCT apparatus. The (modified) planning CT volume is then registered to the CBCT volume with either a rigid or an elastic deformation algorithm (step 232aii). Following registration, the registered CT volume may be processed (for example using a Gaussian filter) to avoid artifacts arising from any remaining discrepancies between the CT and CBCT volumes. Synthetic projection data is then acquired from the registered CT volume using substantially the same acquisition geometry as used for the CBCT scan, with the exception that the simulated detector is made virtually longer than the actual detector (B) (steps 234, 234bi). The patient specific ML model (in this example a neural network) is then trained to predict extended projections from CBCT standard sized projections (step 260). Specifically, during training the input is trimmed or truncated versions of the full synthetic projections (trimmed to the real detector size, C in Figure 6). The target output of the model is the extended region of the full long projection. The loss function for the training compares the predicted extensions with the synthetic projections generated from the planning CT (the full projections in C).

Once trained (D), the neural network is applied to the CBCT projection data of the obtained projection set, producing extended versions of these projections (E) (step 240b, 240bi, 240bia). In order to avoid artifacts in the transition between real and extended CBCT projection data, appropriate normalization and smoothing may be performed on the extended projection data. Finally, the extended real CBCT projection data is used to produce a final reconstruction with extended FOV (steps 150, 250).

It will be appreciated that generating the synthetic projection data from the registered planning CT, and in training the patient specific ML model, suitable augmentations and normalization may be used, to allow the trained network to generalize well to the real CBCT projection data. In some examples, to reduce or even avoid the training of the model after acquisition of the CBCT projections, a model can be pretrained using the isocenter of the treatment plan or with CBCT images from prior fractions to reposition the CT volume (steps 232bi, 234bi). Also, translation and rotation variations of the planning CT volume (obtained 3D image) can be used to augment the training data and make the model more robust against misalignment.

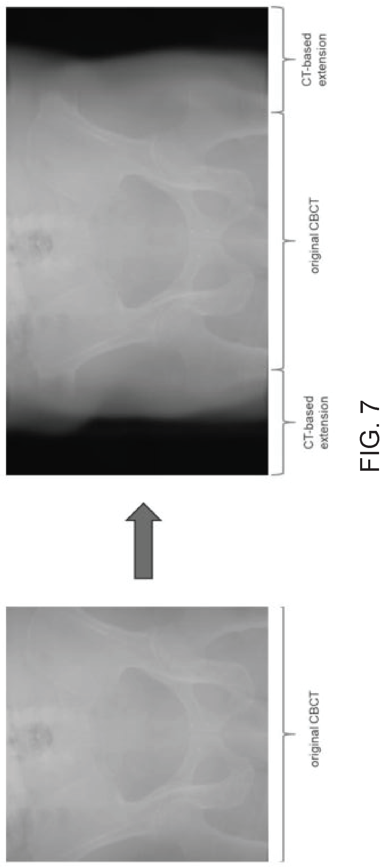

Figure 7 illustrates a comparison between a reconstructed CBCT image, and a reconstructed CBCT image that has been extended according to methods of the present disclosure. On the left of Figure 7 is shown a reconstructed image of a first volume of a patient, the image having been reconstructed from projection data obtained during a CBCT scan. It can be seen that the FOV of the image on the left of Figure 7 is relatively limited, and does not encompass the full patient anatomy in the imaged region. Truncation artefacts may also be present at the edge of the first image. On the right of Figure 7 is shown an image reconstructed from the same CBCT projection data but extended according to examples of the present disclosure. The image on the right of Figure 7 encompasses a combination of the first patient volume and a second patient volume present in the 3D image used to extend the CBCT projections according to the present disclosure. The FOV of the image on the right of Figure 7 is thus considerably larger, with the second patient volume encompassing regions either side of the original FOV. The image on the right of Figure 7 is an illustration of a possible output of the methods disclosed herein.

Example methods according to the present disclosure thus provide an improved method for reconstruction of a three-dimensional image of a patient undergoing radiotherapy treatment. By leveraging prior information about the patient that is present in an obtained 3D image (which may be a planning CT, MRI, or prior CBCT image of the patient), an anatomically correct image with a larger FOV may be constructed. Additionally, by working in projection space, examples of the present disclosure can minimise truncation artefacts, as well as improving the internal consistency of the projection data, and thus improving the resulting reconstruction even within non-truncated regions.

High quality CBCT images are extremely important for radiotherapy applications, as the accuracy of the image impacts the accuracy of the subsequent radiotherapy treatment. By enlarging the FOV of a CBCT image, a larger volume of the patient may be viewed, providing additional options for treatment planning and delivery. Additionally, enlarging the FOV of an image in the manner presented herein may reduce truncation artifacts that appear along the edge of the image, and which occur when parts of the imaged body of the patient remain outside the FOV of the image. This artefact removal improves image quality, enhancing the accuracy of subsequent RT tasks.

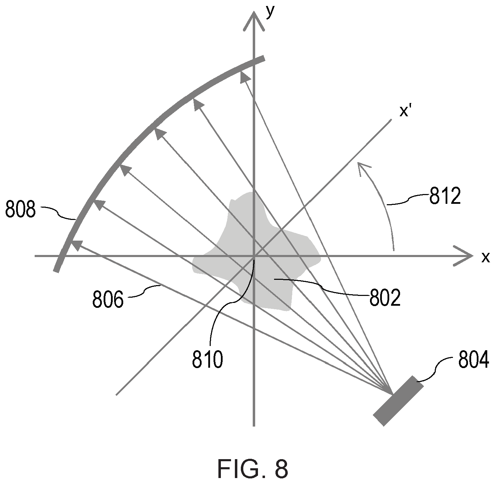

Figure 8 illustrates the geometry of a typical cone beam projection acquisition setup, as may be used in image acquisition and image reconstruction techniques according to techniques of the present disclosure. For simplicity, only the x-y plane is illustrated; although it will be appreciated that the beam of rays extends also into the z-axis, orthogonal to the x-y plane. In this example, the acquisition setup captures projection data of object 802 (which may, for example, be the patient). X-ray source 804 generates and emits X-rays 806 towards object 802. In CBCT, X-rays may be considered as a beam of rays, emitted from a point source. Detector (or detectors) 808 capture projections, which are sets of line integrals along paths that radiate from the source 804. Multiple projections of the image may be acquired from different angles by rotating the source 804 and detector 808 around the centre of the image 810. In the present example, the source 804 and detector 808 may be rotated arcuately along orbital path 812 (referred to herein as a scan arc or CBCT scan arc). In this way, the x-y plane may be rotated counterclockwise around the point of origin (or centre of the image 810) in a manner that keeps the mutual positional relationship between source 804 and detector 808 when passing through the orbital path 812. Other configurations of imaging systems are, of course, feasible; for instance, the source may be configured to rotate and a complete ring of detectors may be configured to capture projections, or there may be multiple sources arranged circumferentially around a complete ring of detectors. Further, the imaging system may rotate the source along a helical path, so as to capture projection data along the axis of the object 802 of interest (along the z-axis in the present example).

The attenuation of the intensity of the rays that pass through the object 802 may be measured by processing signals received from the detector 808. By making projective measurements at a series of different projection angles through the object 802, a sinogram may be constructed from the projection data, mapping the spatial dimension of the detector array to the projection angle dimension. The intensity attenuation resulting from a particular volume within the object will trace out a sine wave for the spatial dimension along the detector perpendicular to the rotation axis of the system. Volumes of the object farther from the centre of rotation correspond to sine waves with greater amplitudes than those corresponding to volumes nearer the centre of rotation. The phase of each sine wave in the sinogram corresponds to the relative angular positions with respect to the rotation axis. By performing an image reconstruction technique (such as an inverse Radon transform) on the projection data in the sinogram, it is possible to reconstruct an image, where the reconstructed image corresponds to a cross-sectional slice or volume of the object 802.

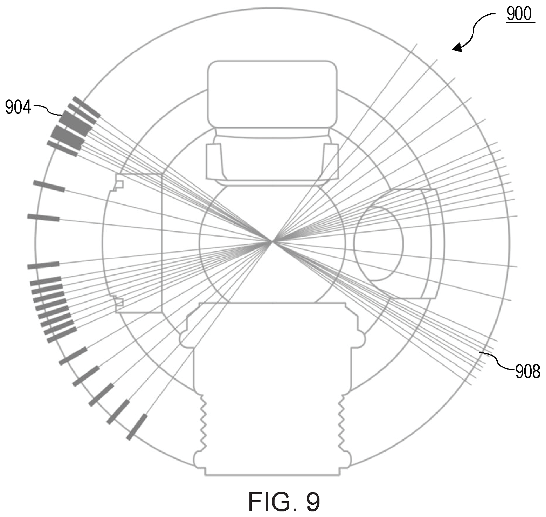

Figure 9 illustrates the geometry of a cone beam projection acquisition setup, as incorporated into a medical CT scanner 900. Radiation sources 904 emit beams of X-ray radiation, which may pass through the patient supported on a couch within the CT scanner (not depicted). Detectors 908 capture attenuated X-rays. The radiation sources 904 and detectors 908 may be configured to rotate within a gantry of the CT scanner, so as to acquire projective measurements at a series of different projection angles.

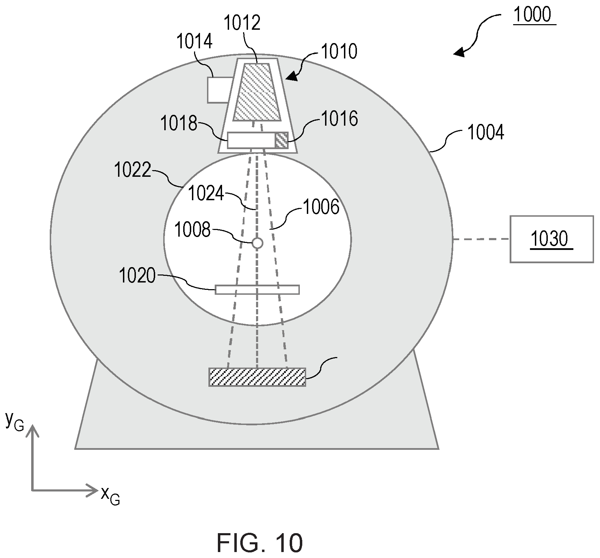

Figure 10 depicts a radiotherapy apparatus, suitable for performing radiotherapy treatment and/or image acquisition. The cross-section through radiotherapy apparatus 1000 includes a radiation head 1010 and a beam receiving apparatus (detector) 1002, both of which are attached to a gantry 1004. The radiation head 1010 includes a radiation source 1012, which emits a beam of radiation 1006. The radiation head 1010 also includes a beam shaping apparatus 1018, which controls the size and shape of the radiation field associated with the beam.

The beam receiving apparatus 1002 is configured to receive radiation emitted from the radiation head 1010, for the purpose of absorbing and/or measuring the beam of radiation. In the view shown, the radiation head 1010 and the beam receiving apparatus 1002 are positioned diametrically opposed to one another.

The gantry 1004 is rotatable, and supports the radiation head 1010 and the beam receiving apparatus 1002 such that they are rotatable around an axis of rotation 1008, which may coincide with the patient longitudinal axis. The gantry provides rotation of the radiation head 1010 and the beam receiving apparatus 1002 in a plane perpendicular to the patient longitudinal axis (e.g., a sagittal plane). Three gantry directions *x_{G}, y_{G}, z_{G}* may be defined such that the *y_{G}* direction is perpendicular with the gantry axis of rotation. The *y_{G}* direction extends from a point on the gantry corresponding to the radiation head 1010, towards the axis of rotation of the gantry. Therefore, from the patient frame of reference, the *y_{G}* direction rotates around as the gantry rotates.

The radiotherapy apparatus 1000 also includes a support surface or couch 1020 on which a subject (or patient) is supported during radiotherapy treatment or image acquisition. The radiation head 1010 is configured to rotate around the axis of rotation 1008 such that the radiation head 1010 directs radiation towards the subject from various angles around the subject in order to spread out the radiation dose received by healthy tissue to a larger region of healthy tissue while building up a prescribed dose of radiation at a target region.

The radiotherapy apparatus 1000 is configured to deliver a radiation beam towards a radiation isocentre, which is substantially located on the axis of rotation 1008 at the centre of the gantry 1004 regardless of the angle at which the radiation head 1010 is placed.

The rotatable gantry 1004 and radiation head 1010 are dimensioned so as to allow a central bore 1022 to exist. The central bore 1022 provides an opening, sufficient to allow a subject to be positioned therethrough without the possibility of being incidentally contacted by the radiation head 1010 or other mechanical components as the gantry rotates the radiation head 1010 about the subject.

The radiation head 1010 emits the radiation beam 1006 along a beam axis 1024 (or radiation axis or beam path), where the beam axis 1024 is used to define the direction in which the radiation is emitted by the radiation head 1010. The radiation beam 1006 is incident on the beam receiving apparatus 1002, which may include at least one of a beam stopper and a radiation detector. The beam receiving apparatus 1002 is attached to the gantry 1004 on a diametrically opposite side to the radiation head 1010 to attenuate and/or detect a beam of radiation after the beam has passed through the subject.

The radiation beam axis 1024 may be defined as, for example, a centre of the radiation beam 1006 or a point of maximum intensity.

The beam shaping apparatus 1018 delimits the spread of the radiation beam 1006. The beam shaping apparatus 1018 is configured to adjust the shape and/or size of a field of radiation produced by the radiation source. The beam shaping apparatus 1018 does this by defining an aperture (also referred to as a window or an opening) of variable shape to collimate the radiation beam 1006 to a chosen cross-sectional shape. In this example, the beam shaping apparatus 1018 may be provided by a combination of a diaphragm and an MLC. Beam shaping apparatus 1018 may also be referred to as a beam modifier.

The radiotherapy apparatus 1000 may be configured to deliver both coplanar and non-coplanar (also referred to as tilted) modes of radiotherapy treatment. In coplanar treatment, radiation is emitted in a plane that is perpendicular to the axis of rotation of the radiation head 1010. In non-coplanar treatment, radiation is emitted at an angle that is not perpendicular to the axis of rotation. In order to deliver coplanar and non-coplanar treatment, the radiation head 1010 may move between at least two positions, one in which the radiation is emitted in a plane which is perpendicular to the axis of rotation (coplanar configuration) and one in which radiation is emitted in a plane which is not perpendicular to the axis of rotation (non-coplanar configuration).

In the coplanar configuration, the radiation head 1010 is positioned to rotate about a rotation axis and in a first plane. In the non-coplanar configuration, the radiation head is tilted with respect to the first plane such that a field of radiation produced by the radiation head is directed at an oblique angle relative to the first plane and the rotation axis. In the non-coplanar configuration, the radiation head 1010 is positioned to rotate in a respective second plane parallel to and displaced from the first plane. The radiation beam is emitted at an oblique angle with respect to the second plane, and therefore as the radiation head rotates the beam sweeps out a cone shape.

In one configuration, the beam receiving apparatus 1002 may remain in the same place relative to the rotatable gantry when the radiotherapy apparatus is in both the coplanar and non-coplanar modes. Therefore, the beam receiving apparatus 1002 is configured to rotate about the rotation axis in the same plane in both coplanar and non-coplanar modes. This may be the same plane as the plane in which the radiation head rotates. In alternative configurations, the beam receiving apparatus 601 may also rotate.

The beam shaping apparatus 1010 is configured to reduce the spread of the field of radiation in the non-coplanar configuration in comparison to the coplanar configuration.

The radiotherapy apparatus 1000 includes a controller 1030, which is programmed to control the radiation source 1012, beam receiving apparatus 1006 and the gantry 1002. Controller 1030 may perform functions or operations such as radiotherapy treatment planning, treatment execution, image acquisition, image processing, motion tracking, motion management, and/or other tasks involved in a radiotherapy process.

Controller 1030 is programmed to control various components of apparatus 1000, such as gantry 1004, radiation head 1010, beam receiving apparatus 1002, and support surface 1020, so as to acquire projection data (i.e., projection images) suitable for image reconstruction.

Hardware components of controller 1030 may include one or more computers (e.g., general purpose computers, workstations, servers, terminals, portable/mobile devices, etc.); processors (e.g., central processing units (CPUs), graphics processing units (GPUs), microprocessors, digital signal processors (DSPs), field programmable gate arrays (FPGAs), special-purpose or specially-designed processors, etc.); memory/storage devices such as a memory (e.g., read-only memories (ROMs), random access memories (RAMs), flash memories, hard drives, optical disks, solid-state drives (SSDs), etc.); input devices (e.g., keyboards, mice, touch screens, mics, buttons, knobs, trackballs, levers, handles, joysticks, etc.); output devices (e.g., displays, printers, speakers, vibration devices, etc.); circuitries; printed circuit boards (PCBs); or other suitable hardware. Software components of controller 1030 may include operation device software, application software, etc.

The radiation head 1010 may be connected to a head actuator 1014, which is configured to actuate the radiation head 1010, for example between a coplanar configuration and one or more non-coplanar configurations, or for example to actuate the radiation source 1012 and/or detector 1002 in response to detection of flex. This may involve translation and rotation of the radiation head 1010 relative to the gantry. In some implementations, the head actuator may include a curved rail along which the radiation head 1010 may be moved to adjust the position and angle of the radiation head 1010. The controller 1030 may control the configuration of the radiation head 1030 via the head actuator 1014.

The beam shaping apparatus 1018 includes a shaping actuator 1016. The shaping actuator is configured to control the position of one or more elements in the beam shaping apparatus 1018 in order to shape the radiation beam 1006. In some implementations, the beam shaping apparatus 1016 includes an MLC, and the shaping actuator 1016 includes means for actuating leaves of the MLC. The beam shaping apparatus 1018 may further comprise a diaphragm, and the shaping actuator 1016 may include means for actuating blocks of the diaphragm. The controller 1030 may control the beam shaping apparatus 1018 via the shaping actuator 1016.

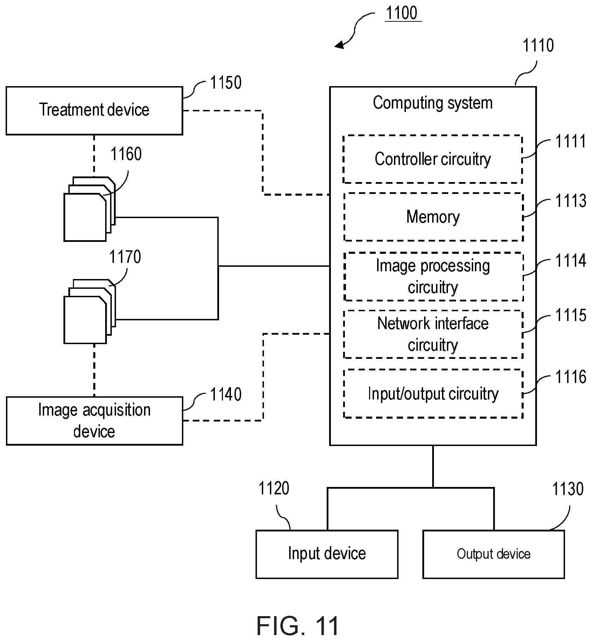

Figure 11 is a block diagram of an implementation of a radiotherapy system 1100, suitable for executing methods for reconstruction of a three-dimensional image of a patient according to examples of the present disclosure. The example radiotherapy system 1100 comprises a computing system 1110 within which a set of instructions, for causing the computing system 1110 to perform the method (or steps thereof) discussed herein, may be executed. The computing system 1110 may implement a CBCT imaging management system. The computing system 1110 may also be referred to as a computer. In particular, the methods described herein may be implemented by a processor or controller circuitry 1111 of the computing system 1110.

The computing system 1110 shall be taken to include any number or collection of machines, e.g., computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 1110 includes controller circuitry 1111 and a memory 1113 (e.g., read-only memory (ROM), flash memory, dynamic random access memory (DRAM) such as synchronous DRAM (SDRAM) or Rambus DRAM (RDRAM), etc.). The memory 1113 may comprise a static memory (e.g., flash memory, static random access memory (SRAM), etc.), and/or a secondary memory (e.g., a data storage device), which communicate with each other via a bus (not shown). Memory 1113 may be used to store or buffer projection data until required for image processing.

Controller circuitry 1111 represents one or more general-purpose processors such as a microprocessor, central processing unit, accelerated processing units, or the like. More particularly, the controller circuitry 1111 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 1111 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 1111 is configured to execute the processing logic for performing the operations and steps discussed herein.

The computing system 1110 may further include a network interface circuitry 1115. The computing system 1110 may be communicatively coupled to an input device 1120 and/or an output device 1130, via input/output circuitry 1116. In some implementations, the input device 1120 and/or the output device 1130 may be elements of the computing system 1110. The input device 1120 may include an alphanumeric input device (e.g., a keyboard or touchscreen), a cursor control device (e.g., a mouse or touchscreen), an audio device such as a microphone, and/or a haptic input device. The output device 1130 may include an audio device such as a speaker, a video display unit (e.g., a liquid crystal display (LCD) or a cathode ray tube (CRT)), and/or a haptic output device. In some implementations, the input device 1120 and the output device 1130 may be provided as a single device, or as separate devices.

In some implementations, the computing system 1110 may comprise image processing circuitry 1114. Image processing circuitry 1114 may be configured to process image data 1170 (e.g., images, imaging data, projections, projection data), such as medical images obtained from one or more imaging data sources, a treatment device 1150 and/or an image acquisition device 1140. Image processing circuitry 1114 may be configured to process, or pre-process, image data 1170. For example, image processing circuitry 1114 may convert received image data into a particular format, size, resolution or the like. Image processing circuitry 1114 may be configured to perform image reconstruction. In some implementations, image processing circuitry 1114 may be combined with controller circuitry 1111.

In some implementations, the radiotherapy system 700 may further comprise an image acquisition device 1140 and/or a treatment device 1150. The image acquisition device 1140 and the treatment device 1150 may be provided as a single device. In some implementations, treatment device 1150 is configured to perform imaging, for example in addition to providing treatment and/or during treatment.

Image acquisition device 1140 may be configured to perform CBCT. Image acquisition device 1140 may be configured to perform positron emission tomography (PET), computed tomography, magnetic resonance imaging (MRI), single positron emission computed tomography (SPECT), X-ray, and the like.

Image acquisition device 1140 may be configured to output image data 1170, which may be accessed by computing system 1110. Treatment device 1150 may be configured to output treatment data 1160, which may be accessed by computing system 1110. Treatment data 1160 may be obtained from an internal data source (e.g., from memory 1113) or from an external data source, such as treatment device 1150 or an external database.

The various methods described above may be implemented by a computer program. The computer program may include computer code (e.g., instructions) arranged to instruct a computer to perform the functions of one or more of the various methods described above. For example, the steps of the methods described in relation to Figure 2 and/or Figures 3a to 3f may be performed by the computer code. The steps of the methods described above may be performed in any suitable order. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions may also reside, completely or at least partially, within the memory 1113 and/or within the controller circuitry 1111 during execution thereof by the computing system 1110, the memory 1113 and the controller circuitry 1111 also constituting computer-readable storage media.

In an implementation, the modules, components and other features described herein may be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components may be implemented as firmware or functional circuitry within hardware devices. Further, the modules and components may be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as "receiving", "determining", "comparing ", "enabling", "maintaining", "identifying", "obtaining", "accessing", or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It should be noted that the above-mentioned examples illustrate rather than limit the disclosure, and that those skilled in the art will be able to design many alternative examples without departing from the scope of the appended claims or numbered examples. The word "comprising" does not exclude the presence of elements or steps other than those listed in a claim or example, "a" or "an" does not exclude a plurality, and a single processor or other unit may fulfil the functions of several units recited in the claims or numbered examples. Any reference signs in the claims or numbered examples shall not be construed so as to limit their scope.

## Claims

1. A computer implemented method for reconstruction of a three-dimensional, 3D, image of a patient, the method comprising:
obtaining a projection set comprising a plurality of two-dimensional, 2D, projections of a first volume of the patient, the projections acquired using a first apparatus which is a Cone-Beam Computed Tomography, CBCT, apparatus;
obtaining a 3D image of a second volume of the patient, the 3D image reconstructed from projections acquired using a second apparatus, wherein the second volume of the patient at least partially overlaps with the first volume of the patient;
using acquisition information relating to the obtained projection set to generate, from the obtained 3D image of the second volume of the patient, synthetic projections of the second volume of the patient;
extending the projections of the first volume of the patient from the obtained projection set with information from the synthetic projections of the second volume of the patient; and
using the extended projections to generate a 3D image of a third volume of the patient, wherein the third volume of the patient comprises a combination of the first volume of the patient and the second volume of the patient.

2. A method as claimed in claim 1, wherein the synthetic projections simulate a longer detector than is present in the first apparatus.

3. A method as claimed in claim 1 or 2, wherein the second apparatus comprises at least one of:
a Fan-Beam Computed Tomography, CT, apparatus;
a Magnetic Resonance Imaging, MRI, apparatus;
a CBCT apparatus.

4. A method as claimed in any one of claims 1 to 3, wherein using acquisition information relating to the projection set to generate, from the obtained 3D image of the second volume of the patient, synthetic projections of the second volume of the patient comprises:
determining an offset between the acquisition geometry of the first apparatus and the acquisition geometry of the second apparatus; and
using the determined offset with the acquisition information relating to the projection set to generate the synthetic projections of the second volume of the patient from the obtained 3D image of the second volume of the patient.

5. A method as claimed in claim 4, wherein determining an offset between the acquisition geometry of the first apparatus and the acquisition geometry of the second apparatus comprises:
reconstructing a 3D image of the first volume of the patient using the obtained projection set; and
registering the obtained 3D image of the second volume of the patient to the reconstructed 3D image of the first volume of the patient.

6. A method as claimed in claim 5, wherein using the determined offset with the acquisition information relating to the projection set to generate the synthetic projections of the second volume of the patient from the obtained 3D image of the second volume of the patient comprises:
using the acquisition information relating to the projection set to generate the synthetic projections of the second volume of the patient from the registered 3D image of the second volume of the patient.

7. A method as claimed in claim 4, wherein determining an offset between the acquisition geometry of the first apparatus and the acquisition geometry of the second apparatus comprises:
using the obtained projection set and the obtained 3D image to calculate an offset between reference features of at least one of:
the first apparatus and the second apparatus;
images reconstructed from projections obtained using the first apparatus and the second apparatus.

8. A method as claimed in claim 7, wherein the reference features comprise one or more of:
isocentres of the first apparatus and the second apparatus;
an anatomical structure of the patient.

9. A method as claimed in claim 7 or claim 8, wherein using the determined offset with the acquisition information relating to the projection set to generate the synthetic projections of the second volume of the patient from the obtained 3D image of the second volume of the patient comprises:
using the calculated offset and the acquisition geometry of the CBCT apparatus to generate the synthetic projections of the second volume of the patient from the obtained 3D image of the second volume of the patient.

10. A method as claimed in any one of the preceding claims, wherein extending the projections of the first volume of the patient from the obtained projection set with information from the synthetic projections of the second volume of the patient comprises:
merging the projections of the first volume of the patient from the obtained projection set with the synthetic projections of the second volume of the patient.

11. A method as claimed in claim 10, wherein merging the projections of the first volume of the patient from the obtained projection set with the synthetic projections of the second volume of the patient comprises:
extending the projections of the first volume of the patient from the obtained projection set using the synthetic projections of the second volume of the patient by intensity matching and stitching the projections of the first volume of the patient from the obtained projection set to the synthetic projections of the second volume of the patient.

12. A method as claimed in any one of the preceding claims, wherein extending the projections of the first volume of the patient from the obtained projection set with information from the synthetic projections of the second volume of the patient comprises:
using a Machine Learning, ML, model to extend the projections of the first volume of the patient from the obtained projection set with information from the synthetic projections of the second volume of the patient, wherein the ML model is operable to receive as input projection data of the projections of the first volume of the patient from the obtained projection set.

13. A method as claimed in claim 12, wherein the ML model is a patient specific model.

14. A method as claimed in claim 13, wherein the patient specific ML model is operable to receive as input projection data of the projections of the first volume of the patient from the obtained projection set, to process the projection data according to trained parameters of the patient specific ML model, and to output projection data of the extended projections.

15. A method as claimed in claim 13 or 14, further comprising:
assembling a training dataset by:
truncating the synthetic projections to remove projection data representing patient anatomy not contained in the first volume of the patient;
adding projection data of the truncated synthetic projections to the training data set as input training data; and
adding projection data of the non-truncated synthetic projections to the training data set as output training data; and
using the training dataset to update trainable parameters of the patient specific ML model.

16. A method as claimed in claim 12, wherein the ML model is a population level model.

17. A method as claimed in claim 16, wherein the population level ML model is operable to receive as input projection data of the projections of the first volume of the patient from the obtained projection set and projection data of the synthetic projections of the second volume of the patient, to process the projection data according to trained parameters of the population level ML model, and to output projection data of the extended projections.

18. A method as claimed in claim 16 or 17, further comprising:
assembling a training dataset by, for individual patients:
adding projection data of projections of the first volume of the patient from the obtained projection set, and projection data of synthetic projections of the second volume of the patient, to the training data set as input training data; and
adding projection data of extended projections to the training data set as output training data; and
using the training dataset to update trainable parameters of the population level ML model.

19. A method as claimed in any one of claims 12 to 18, wherein the ML model is operable to receive as input projection data in the form of at least one of:
individual projections;
a plurality of projections;
a complete projection stack;
sinogram slices from a complete projection stack.

20. A computer program product comprising a computer readable medium, the computer readable medium having computer readable code embodied therein, the computer readable code being configured such that, on execution by a suitable computer or processor, the computer or processor is caused to perform a method as claimed in any one of the preceding claims.

21. A reconstruction node for reconstruction of a three-dimensional, 3D, image of a patient, the reconstruction node comprising processing circuitry configured to cause the reconstruction node to:
obtain a projection set comprising a plurality of two-dimensional, 2D, projections of a first volume of the patient, the projections acquired using a first apparatus which is a Cone-Beam Computed Tomography, CBCT, apparatus;
obtain a 3D image of a second volume of the patient, the 3D image reconstructed from projections acquired using a second apparatus, wherein the second volume of the patient at least partially overlaps with the first volume of the patient;
use acquisition information relating to the obtained projection set to generate, from the obtained 3D image of the second volume of the patient, synthetic projections of the second volume of the patient;
extend the projections of the first volume of the patient from the obtained projection set with information from the synthetic projections of the second volume of the patient; and
use the extended projections to generate a 3D image of a third volume of the patient, wherein the third volume of the patient comprises a combination of the first volume of the patient and the second volume of the patient.

22. A reconstruction node as claimed in claim 21, wherein the processing circuitry is further configured to cause the reconstruction node to carry out a method according to any one of claims 2 to 19.

23. A radiotherapy treatment apparatus comprising a reconstruction node as claimed in claim 21 or 22.
